(19) **Europäisches Patentamt / European Patent Office / Office européen des brevets**

(11) **EP 3 377 882 B1**

(12) **FASCICULE DE BREVET EUROPEEN**

(45) Date de publication et mention de la délivrance du brevet:
**01.01.2025 Bulletin 2025/01**

(21) Numéro de dépôt: **16795352.0**

(22) Date de dépôt: **15.11.2016**

(51) Classification Internationale des Brevets (IPC):
**G01N 27/06** *(2006.01)*  **G01N 33/49** *(2006.01)*

(52) Classification Coopérative des Brevets (CPC):
**G01N 33/4905; G01N 27/06;** G01N 2800/224

(86) Numéro de dépôt international:
**PCT/EP2016/077722**

(87) Numéro de publication internationale:
**WO 2017/085058 (26.05.2017 Gazette 2017/21)**

(54) **METHODE DE DIAGNOSTIC D'ANOMALIES DE LA COAGULATION SANGUINE**

VERFAHREN ZUR DIAGNOSE VON BLUTGERINNUNGSSTÖRUNGEN

METHOD FOR DIAGNOSING BLOOD COAGULATION DISORDERS

(84) Etats contractants désignés:
AL AT BE BG CH CY CZ DE DK EE ES FI FR GB GR HR HU IE IS IT LI LT LU LV MC MK MT NL NO PL PT RO RS SE SI SK SM TR

(30) Priorité: **16.11.2015 FR 1560945**

(43) Date de publication de la demande:
**26.09.2018 Bulletin 2018/39**

(73) Titulaire: **Hospices Civils De Lyon**
**69002 Lyon (FR)**

(72) Inventeur: **NOUGIER, Christophe**
**69190 Saint Fons (FR)**

(74) Mandataire: **Cabinet Nony**
**11 rue Saint-Georges**
**75009 Paris (FR)**

(56) Documents cités:
**WO-A1-2013/143548 US-B2- 8 877 510**

• **HALIMEH S. ET AL.: "Multiplate whole blood impedance point of care aggregometry: Preliminary reference values in healthy infants, children and adolescents", KLIN. PAEDIATR., vol. 222, no. 03, 155, May 2010 (2010-05-01), pages 1 - 6, XP055266006**

• **GÖRLINGER K. ET AL.: "Coagulation management in patients undergoing mechanical circulatory support", BEST PRACT. RES. CLIN. ANESTHESIOL., vol. 26, no. 2, June 2012 (2012-06-01), pages 179 - 198, XP055244717**

• **GERLACH R. ET AL.: "Hemostatic and hemorrhagic problems in neurosurgical patients", ACTA NEUROCHIR., vol. 151, no. 8, 26 June 2009 (2009-06-26), pages 873 - 900, XP019742132**

• **KIND S.L. ET AL.: "Is dilutional coagulopathy induced by different colloids reversible by replacement of fibrinogen and factor XIII concentrates?", ANESTH. ANALG., vol. 117, no. 5, November 2013 (2013-11-01), pages 1063 - 1071, XP009189563**

• **FARAONI D. ET AL.: "Goal-directed coagulation management in the perioperative period of cardiac surgery", J. CARDIOTHORAC. VASC. ANESTH., vol. 27, no. 6, December 2013 (2013-12-01), pages 1347 - 1354, XP028779951**

## Description

La présente invention se rapporte à une nouvelle méthode pour évaluer *ex vivo* le processus de coagulation sanguine, et notamment pour diagnostiquer à partir d'un échantillon de sang issu d'un individu des anomalies de la coagulation, telles que des états d'hémophilie ou autres déficits en facteur de coagulation.

La coagulation sanguine est un processus complexe permettant de combler une brèche au niveau de la paroi endothéliale des vaisseaux sanguins chez les mammifères.

La coagulation, ou hémostase, débute presque instantanément après une lésion de la paroi des vaisseaux sanguins. Elle se décompose en trois étapes principales :

1) Formation d'un « clou plaquettaire » par agrégation des plaquettes, pour bloquer le saignement : c'est l'hémostase primaire.

2) Induction d'une cascade d'événements complexe, lors de laquelle des protéines plasmatiques appelées « facteurs de coagulation » réagissent pour former des fibres de fibrine, qui renforcent le clou plaquettaire : c'est l'hémostase secondaire.

3) Destruction du caillot primaire ou fibrinolyse.

De nombreux troubles de la coagulation, qui mènent à des risques de saignements plus importants, ont été répertoriés. Ces troubles de la coagulation sont liés chacun à un déficit ou une anomalie de l'un des facteurs impliqués dans la cascade, et se manifestent cliniquement par des pathologies hémorragiques qualifiées de mineures, modérées ou sévères, selon la sévérité du déficit ou de l'anomalie.

Les maladies provoquant un problème de coagulation sont appelées les coagulopathies ; parmi celles-ci on distingue les maladies liées à un trouble de l'hémostase primaire, et celles dont l'origine est un défaut des facteurs de coagulation, de façon constitutionnelle ou acquise.

Les maladies de l'hémostase primaire incluent les thrombopénies, les thrombopathies, et la maladie de Willebrand.

Une thrombopénie ou thrombocytopénie est une diminution significative du nombre de plaquettes sanguines en dessous du seuil de 150 000 plaquettes par millimètre cube.

Les thrombopathies sont liées à des dysfonctions plaquettaires diverses. Elles sont le plus souvent acquises, dues à des prises médicamenteuses (aspirine, anti-inflammatoires non stéroïdiens...). Les thrombopathies constitutionnelles, héréditaires, sont beaucoup plus rares.

La maladie de Willebrand est la plus fréquente des pathologies constitutionnelles de l'hémostase primaire. Elle est due à un déficit quantitatif ou qualitatif du facteur de von Willebrand (vWF), qui permet l'adhésion des plaquettes au sous-endothélium.

Les pathologies hémorragiques liées à des facteurs de coagulation sont soit acquises, telles que l'insuffisance hépatocellulaire, les coagulations intravasculaires disséminées, l'hypovitaminose K, et la présence d'anticorps anti-facteur comme dans l'hémophilie acquise ; soit constitutionnelles telles que les hémophilies, le déficit en facteurs de la coagulation XI, VII, XIII, II, X et l'hypofibrinogénémie ou l'afibrinogénémie.

Les hémophilies sont liées à une anomalie constitutionnelle de la coagulation sanguine, en rapport avec un déficit en facteur VIII (Hémophilie A) ou en facteur IX (hémophilie B). L'anomalie génétique responsable est située sur le gène du F8 ou du *F9*. Dans un tiers des cas, l'hémophilie est engendrée par une mutation *de novo* ; dans les autres cas la mutation est transmise de façon héréditaire.

D'autres anomalies constitutionnelles plus rares concernent les déficits en Facteur XI, Facteur VII, Facteur XIII, Facteur X, Facteur II ou encore la faible quantité ou l'absence de fibrinogène. Les anomalies peuvent êtres quantitatives, liées à une absence ou une diminution de concentration de facteur de coagulation, ou qualitatives, dans les cas où le facteur de coagulation est présent mais peu ou non fonctionnel.

Les manifestations cliniques des anomalies constitutionnelles en facteur de coagulation sont principalement les hémorragies, qui peuvent atteindre chaque organe. Elles sont proportionnelles à l'importance du déficit ou défaut du facteur de la coagulation. La maladie peut être sévère avec des manifestations hémorragiques spontanées dès la première année de vie chez un hémophile sévère, ou présenter un tableau clinique plus discret en cas de déficit modéré ou mineur.

Les déficits congénitaux en fibrinogène, très rares, sont des troubles de la coagulation dus à une réduction de la concentration et/ou de la fonction du fibrinogène circulant et caractérisés par des hémorragies de sévérité variable.

D'autres déficits héréditaires en facteur(s) de coagulation ont été décrits mais restent exceptionnels. Dans ces cas également, les manifestations hémorragiques sont variables et dépendent de la sévérité de l'anomalie.

## ART ANTERIEUR

A ce jour, plusieurs tests de dépistage des anomalies de la coagulation du sang sont connus et utilisés en routine par les cliniciens.

Ces tests sont utilisés à des fins diverses, notamment pour le diagnostic des troubles listés ci-dessus, pour suivre l'effet d'un traitement coagulant ou anticoagulant sur un individu, ou encore pour cribler des traitements coagulants ou anticoagulants potentiels.

D'une manière générale, pour les praticiens hospitaliers, il est nécessaire de détecter les anomalies de la coagulation d'un individu avant de pratiquer une intervention chirurgicale sur ledit individu, afin d'exclure le risque hémorragique.

En dehors de la numération plaquettaire, les tests de mesure du temps de Quick (TQ, également connu sous la dénomination taux de prothrombine ou TP) et temps de céphaline + activeur (TCA, aussi appelé TCK si l'activateur utilisé est le kaolin) sont les deux examens les plus fréquemment prescrits pour le dépistage d'une coagulopathie.

Le TQ permet de mesurer le temps de coagulation dans des conditions *in vitro,* après activation par une thromboplastine calcique (contenant du calcium, du facteur tissulaire en forte concentration -de l'ordre de 4 à 6 nM- et des phospholipides pro-coagulants). Le résultat du TQ est compris entre 11 et 13 secondes pour un échantillon de sang normal. Un allongement de cette durée est un indicateur de la présence d'une anomalie de la coagulation, notamment d'un déficit constitutionnel ou acquis en Facteur VII.

Le TCA (Temps de Céphaline Activé) permet de mesurer le temps de coagulation dans des conditions *in vitro,* la céphaline utilisée contenant un activateur et des phospholipides pro-coagulants. Chez l'adulte, la valeur de référence est comprise entre 30 et 40 secondes. Un allongement significatif du TCA est un indicateur de la présence d'une anomalie de la coagulation liée à une hémophilie A ou B, un déficit en facteur XI, XII ou à la prise d'un traitement anticoagulant.

Le test de temps de thrombine (TT) permet de mesurer le temps de coagulation d'un plasma sanguin suite à l'ajout de thrombine et de calcium. La valeur de référence est d'environ vingt secondes. Cet examen teste la transformation du fibrinogène en fibrine, il est utilisé en cas de syndrome hémorragique ou de traitement par l'héparine ou par thrombolytique. Il est aujourd'hui de moins en moins utilisé, sauf pour le diagnostic des défauts en fibrinogène.

Le test de génération de thrombine, une méthode semi-automatisée basée sur l'utilisation d'un substrat chromogène ou fluorogène, permet de mesurer la génération de thrombine *in vitro* après activation avec du facteur tissulaire. Ce test, intéressant car proche des conditions physiologiques, a une mise en oeuvre délicate due à des conditions pré-analytiques spécifiques, un manque de standardisation, et est ainsi limité à des laboratoires expérimentés.

Les inconvénients majeurs de ces tests sont d'une part leur manque de sensibilité pour les pathologies modérées, et d'autre part leur absence de spécificité ; il est souvent nécessaire d'avoir recours à des tests complémentaires (dosages de facteurs, recherche d'anticoagulant circulant...) pour comprendre l'origine du trouble de la coagulation observé.

Ainsi, un « bilan de coagulation » comprend souvent la réalisation de plusieurs tests complémentaires du TQ et du TCA tel que le dosage du fibrinogène, complété en cas d'anomalie par des tests plus spécifiques. Ceci représente un coût élevé à la fois en termes de réactifs et de matériel, ainsi qu'en temps de travail.

Par ailleurs, un inconvénient majeur des tests actuellement mis en oeuvre est qu'ils doivent être réalisées sur du plasma pauvre en plaquette, obtenu à partir d'un échantillon de sang total après 10 à 15 minutes de centrifugation dudit échantillon. Ceci limite la réalisation de ces tests en cas d'urgence en raison du délai supplémentaire dû à cette étape de centrifugation ; et induit un coût supplémentaire lié au pré-traitement de l'échantillon sanguin.

Parmi les tests pouvant être réalisés sur sang total, sans centrifugation, on citera le test du temps de coagulation activé (ACT) ; ce test est peu utilisé car n'est adapté que pour une seule indication, la surveillance de l'héparinothérapie pré-opératoire de chirurgie cardiaque et la neutralisation de celle-ci par la protamine.

On citera également la thromboélastographie en sang total (TEG) qui permet une exploration complète du processus de coagulation, de la formation initiale du caillot jusqu'à sa rétraction et sa dissolution, grâce à un appareillage spécifique. La technique est basée sur le suivi du mouvement d'une goupille animée d'un mouvement de rotation permanent, la coagulation du sang interférant avec la rotation de la goupille. Le thromboélastogramme rotatif (ROTEM®, Allemagne) permet de réaliser en parallèle plusieurs tests différents, par l'utilisation de réactifs spécifiques (Lang et al., 2005).

L'inconvénient de cette technique est la nécessité d'employer de nombreux réactifs, et de devoir procéder à plusieurs tests en parallèle, pour obtenir une vision globale des différentes caractéristiques de la coagulation. Par ailleurs, la sensibilité à certaines anomalies de la coagulation, notamment l'hémophilie, est médiocre.

Actuellement il n'existe pas de test permettant d'identifier un individu atteint d'une coagulopathie, et notamment un individu atteint d'un trouble hémophile, sur la base d'un test unique.

Pour l'hémophilie A ou B, le diagnostic repose en effet, en plus de la symptomatologie clinique, sur la mise en évidence :

- d'un allongement isolé du TCA, sans anticoagulant circulant, associé avec des résultats normaux aux tests TQ ;
- d'un déficit isolé de facteur FVIII ou FIX.

De même, il n'existe pas de test permettant d'identifier un individu présentant un déficit en un autre facteur de la coagulation sur la base d'un test unique.

D'une manière plus générale, il n'existe pas de test global, rapide, pouvant être réalisé sur un faible volume de sang total, sans nécessiter une première étape de centrifugation, et basé sur l'utilisation d'un seul réactif et sur la mesure d'une seule valeur, capable de prédire le risque hémorragique et de dépister les anomalies constitutionnelles ou acquises de la coagulation.

La mise à disponibilité d'un tel test de dépistage simple et rapide devrait permettre un gain de temps et un gain médico-économique dans l'exploration des anomalies de l'hémostase.

**FIGURES**

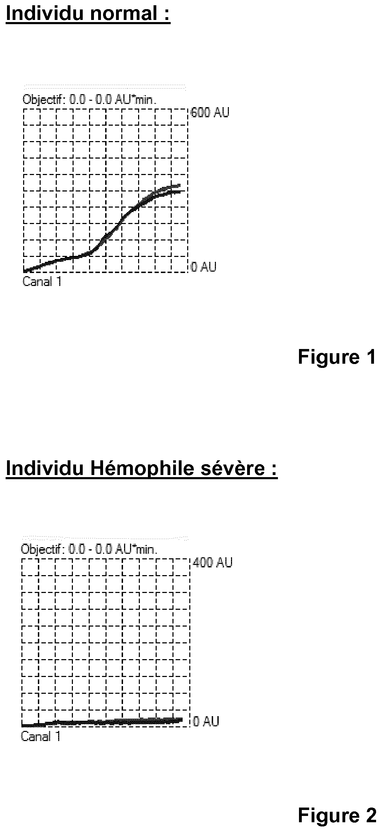

Les figures 1-4 et 6-7 présentent des courbes représentant la mesure de la variation d'impédance au cours du temps, sur des échantillons de sang total, cette mesure étant réalisée par un Multiplate-analyzer®. L'axe des abscisses représente le temps, mesuré à partir de l'ajout du facteur tissulaire, sur une période de 20 minutes. L'axe des ordonnées représente la variation de l'impédance, en unités arbitraires (AU pour Arbitrary Units).

**Figure 1.** Echantillon issu d'un individu ne présentant pas d'anomalie de la coagulation.

Concentration de facteur tissulaire : 2 pM
Aire sous la courbe : 4703 UA*minute

**Figure 2.** Echantillon issu d'un individu hémophile sévère

Concentration de facteur tissulaire : 2 pM
Aire sous la courbe : 301 UA*minute

**Figure 3.** Echantillon issu d'un individu hémophile mineur

Concentration de facteur tissulaire : 2 pM
Aire sous la courbe : 2180 UA*minute

**Figure 4.** Echantillon issu d'un individu présentant un déficit en fibrinogène

Concentration de facteur tissulaire : 2 pM
Aire sous la courbe : 1173 UA*minute

**Figure 5.** Schéma récapitulatif de la répartition des valeurs d'aire sous la courbe, en fonction des populations étudiées. Les valeurs d'aire sous la courbe sont indiquées en ordonnées en AUC (AU*minute).
Les populations représentées sont les suivantes : individus témoins, hémophiles A (HA) et B (HB) sévères, hémophiles A (HA) et B (HB) modérés, hémophiles A (HA) et B (HB) mineurs, individu avec une hypofibrinigénémie, individu présentant un déficit mineur en facteur XI.

**Figure 6.** Test avec une concentration de facteur tissulaire : 100 pM

A) Echantillon issu d'un individu ne présentant pas d'anomalie de la coagulation.
B) Echantillon issu d'un individu hémophile

**Figure 7.** Test avec une concentration de facteur tissulaire : 1 pM

A) Echantillon issu d'un individu ne présentant pas d'anomalie de la coagulation.
B) Echantillon issu d'un individu hémophile

**Figure 8.** Courbe de la corrélation entre le taux d'activité de facteur VIII plasmatique (en abscisse) et l'aire sous la courbe mesurée chez les individus (en ordonnées)

Le taux d'activité du facteur VIII plasmatique est inférieur à 1% pour les hémophiles A sévères, compris entre 1 et 5% pour les hémophiles A modérés, et compris entre 5 et 40% pour les hémophiles A mineurs. Les valeurs au-dessus de 40% ont été déterminées sur des échantillons d'individus non hémophiles.

La corrélation observée est excellente : r2= 0,883.

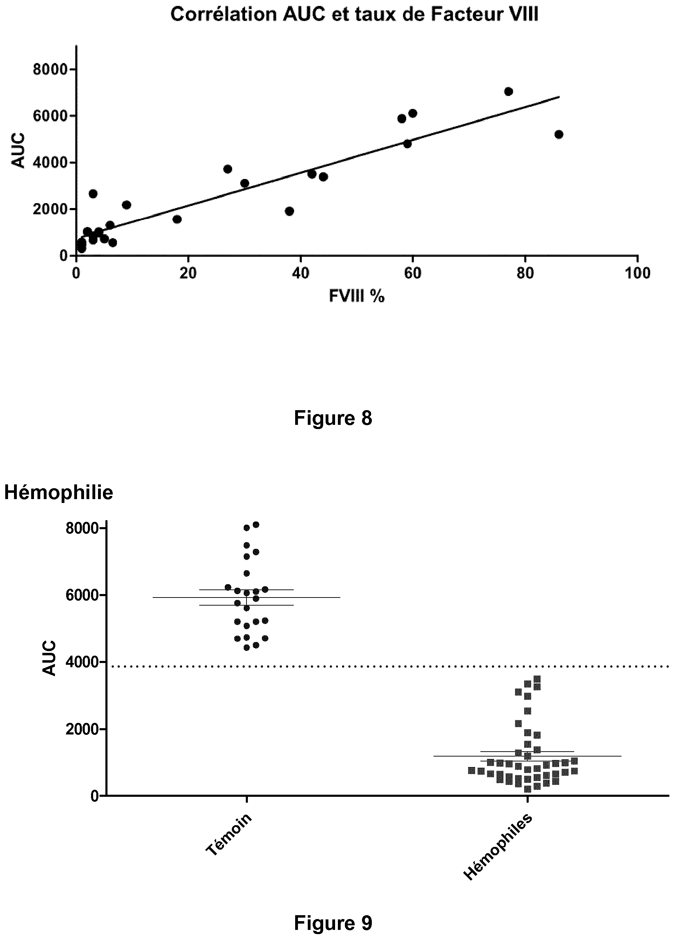

**Figure 9.** Schéma récapitulatif de la répartition des valeurs d'aire sous la courbe, en fonction des populations étudiées. Les valeurs d'aire sous la courbe sont indiquées en ordonnées en AUC (AU*minute).

Les populations représentées sont les suivantes : individus témoins et individus hémophiles, tout type d'hémophilie et de sévérité confondus. la valeur d'AUC moyenne des témoins est 5930 ; celle des hémophiles est de 1192. L'analyse statistique est très significative (p<0,0001).

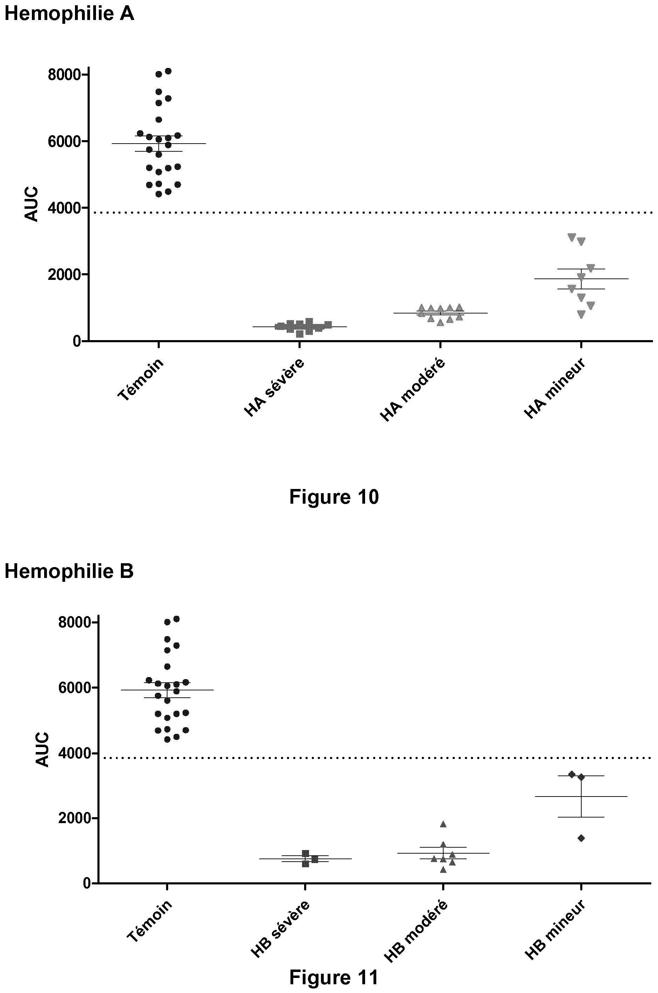

**Figure 10.** Schéma récapitulatif de la répartition des valeurs d'aire sous la courbe, en fonction des populations étudiées pour le diagnostic de l'hémophilie A (HA). Les valeurs d'aire sous la courbe sont indiquées en ordonnées en AUC (AU*minute).

Les populations représentées sont les suivantes : individus témoins (effectif : 23) ; individus présentant une HA sévère (effectif : 9) ; individus présentant une HA modéré (effectif : 10) et individus présentant une HA mineure (effectif : 8).

**Figure 11.** Schéma récapitulatif de la répartition des valeurs d'aire sous la courbe, en fonction des populations étudiées pour le diagnostic de l'hémophilie B (HB). Les valeurs d'aire sous la courbe sont indiquées en ordonnées en AUC (AU*minute).

Les populations représentées sont les suivantes : individus témoins (effectif : 23) ; individus présentant une HB sévère (effectif : 3) ; individus présentant une HB modéré (effectif : 7) et individus présentant une HB mineure (effectif : 3).

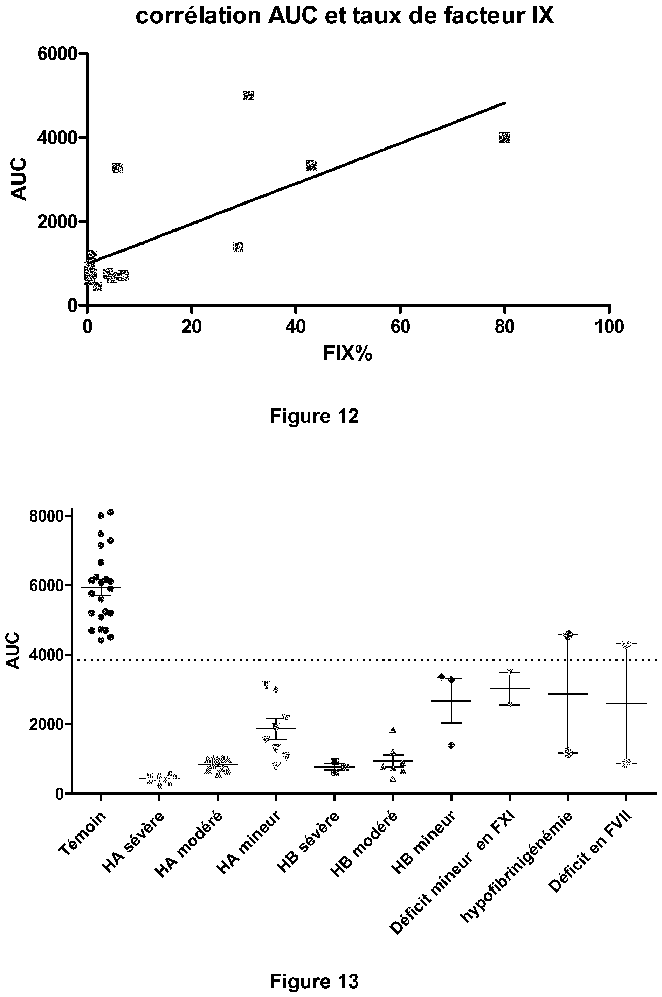

**Figure 12.** Courbe de la corrélation entre le taux d'activité de facteur IX plasmatique (en abscisse) et l'aire sous la courbe mesurée chez les individus (en ordonnées)

Le taux d'activité du facteur IX est inférieur à 1% pour les hémophiles B sévères, compris entre 1 et 5% pour les hémophiles B modérés, et compris entre 5 et 40% pour les hémophiles B mineurs. Les valeurs au-dessus de 40% ont été déterminées sur des échantillons d'individus non hémophiles.

La corrélation observée est bonne : r2=0,5417.

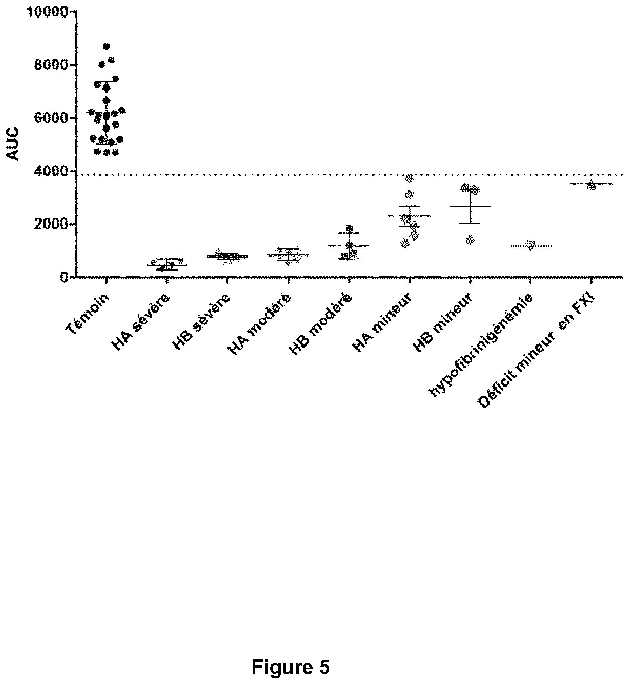

**Figure 13.** Schéma récapitulatif de la répartition des valeurs d'aire sous la courbe, en fonction des populations étudiées. Les valeurs d'aire sous la courbe sont indiquées en ordonnées en AUC (AU*minute). Les effectifs de chaque population sont supérieurs à ceux de la figure 5.

Les populations représentées sont les suivantes : individus témoins, hémophiles A (HA) et B (HB) sévères, hémophiles A (HA) et B (HB) modérés, hémophiles A (HA) et B (HB) mineurs, individus présentant un déficit mineur en facteur XI, individus présentant une hypofibrinigénémie, individus présentant un déficit en facteur VII.

## RESUME DE L'INVENTION

La présente invention concerne une méthode pour diagnostiquer des anomalies de la coagulation sanguine, basée sur la mesure de l'impédance dans un échantillon de sang total, après ajout de facteur tissulaire en une concentration adéquate.

La présente méthode se base sur la détermination d'une valeur d'aire sous la courbe, la courbe représentant la variation d'impédance mesurée entre deux électrodes immergées dans une solution de sang total, dont la coagulation est déclenchée par ajout de facteur tissulaire en faible concentration, au cours du temps.

La détermination de cette valeur permet de déterminer de façon rapide, précise et spécifique si un individu présente une anomalie de la coagulation, et si celle-ci est sévère ou modérée.

La présente invention présente les avantages suivants :

- cette méthode de diagnostic s'effectue sur du sang total, elle est donc rapide puisqu'elle ne nécessite pas de centrifugation de l'échantillon de sang ;
- cette méthode permet de diagnostiquer, par un seul test, différentes anomalies de la coagulation sanguine, et notamment des anomalies de l'hémostase secondaire ;
- cette méthode ne nécessite pas un appareillage supplémentaire à celui déjà existant ; elle peut notamment être réalisée sur des appareillages déjà utilisés en routine, tel que le Multiplate-analyzer® ;
- cette méthode est réalisée sur des échantillons de faible volume, notamment inférieurs à 1 ml, réduisant ainsi le volume de prélèvement nécessaire, ce qui est particulièrement avantageux lorsque les individus sont des enfants.

La mise en oeuvre de la présente méthode permet notamment de diagnostiquer, en un test unique, des troubles de l'hémostase secondaire, et notamment les hémophilies.

La présente invention est également relative à une méthode pour effectuer un suivi thérapeutique personnalisé d'individus atteints d'une anomalie de la coagulation, et notamment d'une méthode pour déterminer l'efficacité d'un traitement d'une anomalie de la coagulation sanguine chez un individu, comprenant les étapes suivantes :

i) déterminer la valeur de l'aire sous la courbe d'un échantillon de sang dudit individu avant le début dudit traitement, et

ii) déterminer la valeur de l'aire sous la courbe d'un échantillon de sang dudit individu après le début dudit traitement,

ledit traitement étant jugé comme efficace lorsque la valeur de l'aire sous la courbe déterminée à l'étape ii) est supérieure à la valeur de l'aire sous la courbe déterminée à l'étape i).

## DESCRIPTION DETAILLEE DE L'INVENTION

Il est entendu que la présente méthode peut être mise en oeuvre sur tout type d'équipement ou d'appareil comprenant *a minima* une cuve, deux paires d'électrodes pouvant être immergées dans la cuve, et un appareil générant un signal électrique et capable de mesurer la variation d'impédance entre les électrodes.

Avantageusement, cet appareil permet également de générer automatiquement une courbe des valeurs d'impédance en fonction du temps.

Selon un aspect préféré de l'invention, la mise en oeuvre de la méthode selon l'invention est réalisée sur un Multiple Analyzer®.

### Multiplate Analyzer®

Il existe des appareils permettant, par la présence de deux paires d'électrodes dans la cuvette contenant l'échantillon de sang, de mesurer l'impédance entre les électrodes, celle-ci étant fonction de la viscosité de l'échantillon et donc de l'avancement du processus de coagulation. Un de ses appareils est en particulier le Multiplate-analyzer®, aussi appelé Multiplate®, commercialisé par ROCHE.

Le Multiplate® se compose de cuvettes à usage unique dans lesquelles sont introduites un échantillon sanguin et un réactif. La cuvette est introduite dans la machine, puis un courant électrique traverse la solution en contact avec les électrodes. L'impédance ainsi mesurée est liée à l'agrégation plaquettaire de l'échantillon de sang, et varie en fonction de celle-ci.

Cet équipement est décrit de manière détaillée notamment dans les demandes de brevet US 8,877,510 et EP 2 182 345.

Jusqu'à présent, les analyses suivantes étaient réalisées avec le Multiplate® :

**Tableau 1**

| Nom de code du test | Description complète du test | Finalité diagnostique du test |
|---|---|---|
| ADP+PGE 1 =ADPtest HS | Agrégation induite par l'ADP et spécifique des récepteurs P2Y12 qui sont la cible des thiénopyridines (inhibition des autres cibles de l'ADP par PGE1) | Dépistage de résistances aux AAP (antiagrégants plaquettaires) |
| ASPItest | Agrégation dépendante des cyclooxygénases, pouvant être inhibées par l'aspirine ou autres AINS | |
| TRAPtest | Stimulation de l'agrégation via les récepteurs de la thrombine. Sensible aux inhibiteurs de GpIIbIIIa | |
| COLLtest | Stimulation de l'agrégation par le collagène | Dépistage d'anomalies de l'hémostase primaire |
| ADPtest | Stimulation de l'agrégation par l'ADP | |
| RISTOhigh | Agrégation dépendante du facteur de Willebrand et du récepteur plaquettaire GpIbIX | |
| RISTOlow | Agrégation dépendante du facteur de Willebrand et du récepteur plaquettaire GpIbIX : détection d'une hyperagrégabilité avec de faibles concentrations en ristocétine | |

Comme indiqué dans ce tableau 1, les indications reconnues de l'analyseur Multiplate® concernent des tests permettant de mesurer l'agrégation plaquettaire c'est-à-dire d'évaluer l'hémostase primaire. L'équipement est en particulier utilisé pour les tests de résistance aux antiagrégants plaquettaires (AAP), pour le dépistage de la maladie de Willebrand, et pour le dépistage des thrombopathies constitutionnelles (Glanzmann, Jean Bernard Soulier, anomalies de sécrétion).

La présente invention est relative à une nouvelle utilisation de cet équipement pour effectuer un test global de dépistage des troubles de l'hémostase, notamment dans le but de diagnostiquer des troubles de l'hémostase secondaire et/ou de la fibrinolyse.

Ainsi, la présente invention est relative à une nouvelle utilisation d'un appareil adapté pour mesurer la variation d'impédance dans un échantillon de sang total, pour diagnostiquer des troubles de l'hémostase secondaire et/ou de la fibrinolyse.

Plus particulièrement, la présente invention concerne une méthode pour diagnostiquer des anomalies de la coagulation sanguine, comprenant les étapes successives suivantes :

a) placer un échantillon de sang total dans une cuve contenant deux paires d'électrodes connectées à un appareil générant un courant électrique ;

b) incuber cet échantillon pendant 60 à 180 secondes en présence de calcium ;

c) ajouter à cet échantillon du facteur tissulaire en une concentration adéquate pour déclencher la coagulation sanguine ;

d) mesurer la variation d'impédance entre les électrodes en fonction du temps, pendant une période comprise entre 10 et 30 minutes à partir de l'étape (c) et générer une courbe des valeurs d'impédance en fonction du temps;

e) comparer la valeur de l'aire sous la courbe générée à l'étape (d) avec une valeur d'aire sous la courbe de référence.

### Conditions optimales de mise en œuvre

La présente invention est basée sur l'utilisation d'un appareil générant un courant électrique, permettant de mesurer la variation d'impédance entre des électrodes immergées dans un échantillon de sang total *ex vivo.*

L'impédance électrique mesure l'opposition d'un circuit électrique au passage d'un courant alternatif sinusoïdal. La variation d'impédance est aussi appelée principe de Coulter, du nom de son inventeur. Elle correspond à la résistance électrique de l'échantillon sanguin, celle-ci variant au cours du processus de coagulation.

Au sens de la présente invention, les termes 'cupule', 'cuvette' et 'cuve' sont utilisés indifféremment pour désigner le récipient dans lequel est introduit l'échantillon de sang total, et dans lequel la mesure de la variation d'impédance est effectuée.

Il est entendu que, dans la mise en oeuvre de la méthode selon l'invention, l'échantillon de sang testé est maintenu à une température d'environ 37°C afin que la coagulation puisse s'effectuer dans des conditions au plus près des conditions physiologiques.

La présence d'un agitateur dans la cuve est optionnelle, mais est préférable pour la mise en oeuvre de l'invention.

Par ailleurs, et comme cela est bien connu de l'homme du métier, la présence de calcium dans l'échantillon sanguin est nécessaire pour la mise en oeuvre de la méthode.

Le calcium joue un rôle indispensable dans la cascade de la coagulation et notamment dans la formation du clou plaquettaire. En effet, dans les plaquettes, le calcium active la protéine kinase C, qui active à son tour la phospholipase A2, qui modifie l'intégrine glycoprotéique IIb/IIIa, augmentant son affinité pour le fibrinogène. Les plaquettes ainsi activées changent de forme: de sphériques, elles deviennent stellaires, et le fibrinogène entrecroisant les glycoprotéines IIb/IIIa promeut l'agrégation des plaquettes. Le calcium permet également la liaison des facteurs de coagulation aux surfaces phospholipidiques exprimées à la surface des plaquettes activées lors du processus de coagulation.

Selon un aspect préféré de l'invention, une solution saline comprenant du calcium est ajoutée à l'échantillon à l'étape (b), de préférence en effectuant une dilution volume / volume.

Cette solution sera choisie parmi les solutions salines contenant du calcium bien connues de l'homme du métier, telles qu'une solution de NaCl/CaCl2 ou une solution de PBS supplémenté en calcium.

A l'étape (b) du procédé, l'échantillon est incubé pendant 60 à 180 secondes, de préférence de 90 à 150 secondes, et plus préférentiellement pendant 120 secondes. Cette « pré-incubation » permet un mélange homogène de l'ensemble des acteurs de la coagulation, et une stabilisation de la température (37°C) de l'échantillon de sang déposé dans la cupule.

### Facteur tissulaire

Les précédentes mesures réalisées avec l'analyseur Multiplate® étaient effectuées après déclenchement du processus d'agrégation grâce à des composés choisis notamment parmi l'acide arachidonique (ASPI), le TRAP (Thrombin receptor-activating peptide), le collagène ou de l'adénosine diphosphate (ADP).

Lors de la mise en oeuvre de la méthode selon l'invention, la réaction de coagulation est déclenchée par l'ajout de facteur tissulaire à faible concentration.

Le facteur tissulaire ajouté au sang total, en présence de calcium, déclenche la génération des premières traces de thrombine. La thrombine générée active les plaquettes contenues dans le sang ; celles-ci vont s'agréger au niveau des électrodes, ce qui entraine une modification du signal électrique entre les électrodes. La coagulation qui suit l'agrégation plaquettaire permet de générer un réseau de fibrine qui va également modifier le signal entre les deux électrodes. La mesure d'impédance en fonction du temps est représentée par une courbe réactionnelle.

La méthode selon l'invention comprend l'addition, à l'étape (c), du facteur tissulaire en une concentration adéquate pour déclencher la coagulation sanguine.

Au sens de la présente invention, l'expression « une concentration adéquate pour déclencher la coagulation sanguine » désigne une concentration finale de facteur tissulaire ajouté dans la cuve permettant de déclencher le processus de coagulation.

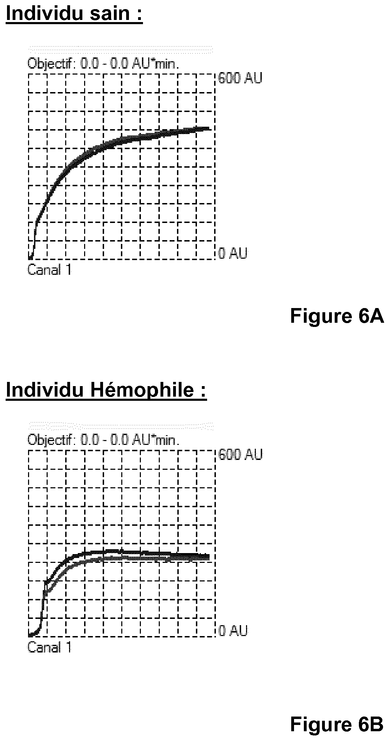

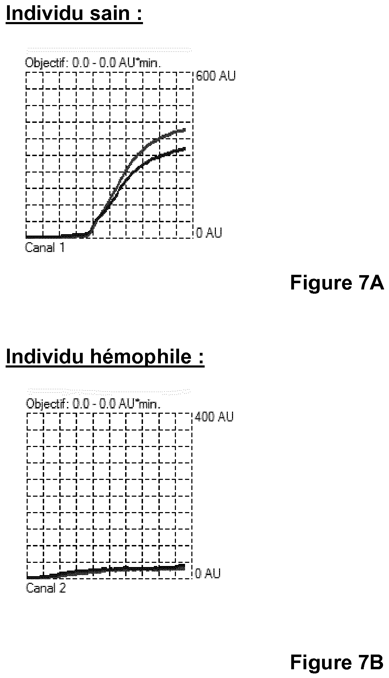

L'exemple 3 présenté ci-après montre les résultats obtenus avec l'ajout de deux concentrations différentes: 100 pM et 1 pM de facteur tissulaire (figures 6A, 6B, et 7A, 7B respectivement). Ces deux concentrations sont adéquates pour déclencher la coagulation sanguine dans l'échantillon de sang testé.

Il a été observé que le signal obtenu, à savoir la valeur de l'aire sous la courbe, est proportionnelle à la concentration utilisée. Il a ainsi été observé que lorsque le facteur tissulaire est ajouté en une concentration finale de 1 pM, les résultats obtenus avec un échantillon issu d'un individu sain, et un échantillon d'un individu hémophile, sont plus nettement distinguables qu'avec des concentrations plus fortes (figures 7A et 7B).

Selon une mise en oeuvre préférée de l'invention, le facteur tissulaire est ajouté à une concentration finale dans l'échantillon comprise entre 0.5 et 5 pM, étant entendu que les bornes de l'intervalle indiqué sont comprises dans la gamme des concentrations préférées.

Le facteur tissulaire pourra en particulier être utilisé à une concentration de 0.5 pM, 0.8 pM, 1 pM, 1.5 pM, 2 pM, 2.5 pM, 3 pM, 3.5 pM, 4 pM, 4.5 pM, ou 5 pM.

Selon un aspect préféré le facteur tissulaire est ajouté en une concentration finale dans l'échantillon de 1 pM ou 2 pM.

Dans d'autres tests de coagulation, tel que le test TQ, le facteur tissulaire est utilisé à des concentrations d'environ 4 nM, soit une concentration largement supérieure à celle utilisée dans la méthode selon la présente invention.

Dans la mise en oeuvre de la présente invention, le facteur tissulaire est ajouté en une concentration relativement plus faible que celle utilisée dans d'autres tests, mais néanmoins adéquate pour déclencher la coagulation sanguine.

### Temps de mesure

La méthode est mise en oeuvre en mesurant la variation d'impédance de l'échantillon de sang en présence de facteur tissulaire, à partir de l'ajout de ce facteur tissulaire, pendant une période comprise entre 10 et 30 minutes, de préférence entre 15 et 25 minutes. Cette période de mesure, plus longue que celles réalisées précédemment avec l'appareil Multiplate®, permet d'obtenir des valeurs d'aire sous la courbe plus élevées et donc permettant de distinguer plus nettement les valeurs observées des échantillons obtenus à partir d'individus présentant une anomalie de la coagulation, de celles observées des échantillons obtenus chez des individus sains.

Selon une mise en oeuvre préférée de l'invention, la mesure de la variation d'impédance est réalisée pendant une période d'environ 20 minutes.

En particulier, les inventeurs ont observé qu'au-delà de 30 minutes les résultats n'étaient plus exploitables, car l'échantillon de sang coagule même en absence de facteur tissulaire.

### Echantillon de sang

Les échantillons de sang humain sont obtenus selon les techniques habituelles, par des expérimentateurs qualifiés. De tels prélèvements sont en particulier réalisés au sein de laboratoires d'analyses médicales, dans des hôpitaux ou cliniques, ou en ambulatoire, notamment dans des unités mobiles de santé.

On entend par « sang total » le sang qui n'a pas été centrifugé et qui contient donc toutes les cellules, notamment les globules rouges, présents dans le sang.

Le sang prélevé est introduit dans des tubes comprenant des composés anticoagulants réversibles, permettant de maintenir la dilution du sang hors de l'organisme, tels que du citrate ou du CTI (Corn Trypsin Inhibitor).

Selon un aspect particulier de l'invention, l'échantillon de sang est dit « citraté » c'est-à-dire qu'il comprend du citrate à titre d'anti-coagulant réversible.

Selon une mise en oeuvre préférée de la méthode selon l'invention, l'échantillon de sang total est citraté.

Selon une autre mise en oeuvre de l'invention, le volume de l'échantillon de sang total est inférieur à 1 ml, de préférence inférieur à 500 µl. L'échantillon testé peut en particulier être d'un volume d'environ 300 µl.

*Courbe de référence*

Il est entendu que la détermination d'une courbe de référence indiquant la réponse normale attendue d'un échantillon de sang à l'ajout de facteur tissulaire, reflétant un processus de coagulation normal, est essentielle à la mise en oeuvre de la méthode selon l'invention.

Cette courbe de référence peut être aisément déterminée par l'homme du métier à l'aide de ses connaissances générales. En effet, une telle courbe est réalisée à partir d'échantillons de sang issus d'individu ne présentant pas de troubles de la coagulation sanguine, ou au contraire présentant un trouble spécifique et connu de la coagulation sanguine .

Selon une mise en oeuvre particulière de l'invention, la courbe de référence est une courbe moyenne obtenue en réalisant les étapes (a) à (d) de la méthode sur des échantillons de sang issus d'individus ne présentant pas d'anomalie de la coagulation, et en calculant la moyenne arithmétique de chacune des valeurs obtenues à l'étape (d).

Selon une autre mise en oeuvre de l'invention, la courbe de référence est la courbe obtenue en réalisant les étapes (a) à (d) de la méthode sur un échantillon de sang issu d'un seul individu ne présentant pas d'anomalie de la coagulation.

En particulier, chaque mesure peut être réalisée en duplicat dans une même cuve réactionnelle, et la moyenne arithmétique de chaque mesure est le chiffre considéré pour dessiner la courbe de référence.

Selon une autre mise en oeuvre de l'invention, la courbe de référence est la courbe obtenue en réalisant les étapes (a) à (d) de la méthode sur un échantillon de sang issu d'un seul individu affecté d'une anomalie de la coagulation déterminée, telle qu'une anomalie « mineure », « modérée » ou « sévère ».

Selon une autre mise en oeuvre de l'invention, la courbe de référence est la courbe obtenue en réalisant les étapes (a) à (d) de la méthode sur des échantillons de sang issus d'individus affectés d'une anomalie de la coagulation déterminée, telle qu'une anomalie « mineure », « modérée » ou « sévère », et en calculant la moyenne arithmétique de chacune des valeurs obtenues à l'étape (d).

A partir de ces courbes de référence, la valeur de l'aire sous la courbe de référence est aisément déterminable, en calculant la surface délimitée par la courbe et par l'axe des abscisses. La valeur numérique de cette « aire sous la courbe » est comparée avec l'aire mesurée sous la courbe de référence, aussi appelée « valeur de référence ».

Une valeur moyenne de référence peut être définie, cette valeur « seuil » séparant sans ambiguïté les valeurs obtenues à partir d'échantillons de sang d'individus témoins, sans anomalie de la coagulation, et ceux d'individus présentant une coagulopathie.

Cette valeur de référence « seuil », aussi dite de « cut-off », est déterminée à partir (i) de valeurs d'aires sous la courbe déterminées chez des individus sans coagulopathie et (ii) de valeurs d'aires sous la courbe déterminées chez des individus présentant une coagulopathie.

Cette valeur d'aire sous la courbe étant basée sur des unités arbitraires, elle pourra être définie de manière chiffrée, sans difficultés, par l'Homme du métier lors de la mise en oeuvre de l'invention.

L'étape (e) de la méthode consiste à comparer la valeur de l'aire sous la courbe générée à l'étape précédente avec la valeur de l'aire sous la courbe de référence.

Selon une mise en oeuvre particulière de l'invention, la variation d'impédance est mesurée une fois par minute pour générer la courbe.

Avantageusement, la variation d'impédance est mesurée une fois toutes les secondes, une fois toutes les deux secondes, une fois toutes les 5 secondes, une fois toutes les dix secondes, une fois toutes les 15 secondes, une fois toutes les 20 secondes, une fois toutes les 30 secondes ou une fois par minute.

La variation d'impédance étant mesurée à l'aide de valeurs arbitraires, chaque expérimentateur déterminera une courbe de référence et une valeur de référence dépendantes du type d'appareillage utilisé pour réaliser la méthode selon l'invention.

D'une manière générale, il peut être défini la relation suivante : si la valeur de l'aire sous la courbe d'un échantillon est inférieure ou égale à la valeur de l'aire sous la courbe de référence, la variation calculée étant d'au moins deux écart-types, il peut être conclu que le individu dont est issu l'échantillon présente probablement une coagulopathie.

Chaque individu étant unique, il est entendu que les valeurs observées d'aires sous la courbe pourront varier légèrement de la valeur de référence, d'une variation inférieure ou égale à un écart-type, sans que cela soit lié à une anomalie de la coagulation.

Au sens de l'invention, un écart-type est défini comme étant égal à la racine carré de la variance, elle-même calculée selon la formule mathématique suivante :

$$\text{Variance} = \text{moyenne } (vi^2) - moy^2,$$

avec vi représentant chaque valeur d'aire sous la courbe, pour chaque échantillon, et moy représentant la moyenne arithmétique des valeurs d'aire sous la courbe.

Les valeurs d'aires sous la courbe obtenues à partir d'échantillons issus de différentes populations d'individus ont été reportées en figures 5 et 13. Sur ces figures, chaque valeur individuelle d'AUC est représentée par un point ; la moyenne des valeurs obtenues dans chaque groupe d'individus est représentée par un trait horizontal ; deux autres traits plus courts, au dessus et en dessous de la moyenne, représentent l'écart type observé autour de cette moyenne de valeurs.

Il apparait clairement que les échantillons de sang issus d'individus présentant des déficits sévères présentent des valeurs d'aire sous la courbe bien inférieures à celles observées pour les échantillons de sang issus d'individus présentant des déficits mineurs, et largement inférieures aux valeurs observées pour les échantillons de sang issus de la population témoin, ne souffrant pas d'anomalies de la coagulation.

### Troubles de l'hémostase et suivi de traitements coagulants

La méthode selon l'invention permet de diagnostiquer des anomalies de la coagulation sanguine.

Avantageusement, la méthode selon l'invention permet également de déterminer la sévérité de l'anomalie de la coagulation sanguine, et de distinguer des cas sévères et des cas mineurs.

La classification des hémophilies selon leur gravité est indiquée dans le tableau 2 ci-dessous :

**Tableau 2**

|  | Classification | Activité coagulante | Manifestations hémorragiques |
|---|---|---|---|
| HEMOPHILIE | Sévère | <1% de l'activité normale | Hémorragies fréquentes, parfois spontanées, à localisations principalement articulaires et musculaires |
|  | Modérée | 1-5% % de l'activité normale | Hémorragies occasionnelles, notamment lors de traumatismes ou d'interventions chirurgicales |
|  | Mineure | >5-40% % de l'activité normale | Hémorragies lors de traumatismes importants ou d'interventions chirurgicales |

Ainsi, l'invention se rapporte également à une méthode pour déterminer la sévérité d'une anomalie de la coagulation chez un individu, comprenant les étapes suivantes :

a) placer un échantillon de sang total dans une cuve contenant deux paires d'électrodes connectées à un appareil générant un courant électrique ;

b) incuber cet échantillon pendant 60 à 180 secondes en présence de calcium ;

c) ajouter à cet échantillon du facteur tissulaire en une concentration adéquate pour déclencher la coagulation sanguine ;

d) mesurer la variation d'impédance entre les électrodes en fonction du temps, pendant une période comprise entre 10 et 30 minutes à partir de l'étape (c) et générer une courbe des valeurs d'impédance en fonction du temps;

e) comparer la valeur de l'aire sous la courbe générée à l'étape (d) avec des valeurs de référence déterminées chez des individus présentant une coagulopathie mineure, modérée, ou sévère.

Les valeurs de référence sont des valeurs d'aire sous la courbe déterminées chez des individus atteints d'une anomalie de la coagulation sévère, mineure ou modérée.

Il est ainsi possible de déterminer la sévérité de l'anomalie de la coagulation observée chez l'individu testé.

De manière avantageuse, les anomalies de la coagulation diagnostiquées sont des troubles de l'hémostase secondaire, pour lesquels il n'existait pas, à ce jour, de test unique. Le diagnostic ne pouvait être posé qu'après de multiples tests et dosages avant de conclure à la présence d'une telle anomalie.

Par troubles de l'hémostase secondaire, on désigne des troubles liés à des déficits ou défauts de facteurs de coagulation ; ces troubles peuvent être constitutionnels ou acquis.

Ainsi, les troubles de l'hémostase secondaire pouvant être diagnostiqués par la méthode selon l'invention sont en particulier choisis parmi :

- les coagulopathies constitutionnelles : Hémophilie A ou B, déficit en d'autres facteurs de la coagulation tel que déficit en facteur XI, VII, XIII, X, V, II et fibrinogène et

- les coagulopathies acquises, dues à la prise médicamenteuse d'inhibiteurs de la coagulation, notamment dans le cadre d'un traitement anticoagulant.

Selon un aspect particulier de l'invention, les anomalies de la coagulation diagnostiquées sont des troubles de la fibrinolyse, qui correspond à l'étape finale de la cascade de coagulation.

Selon un aspect particulier de l'invention, les anomalies de la coagulation pouvant être diagnostiquées par le procédé selon l'invention sont des troubles de l'hémostase secondaire et/ou de la fibrinolyse.

La méthode selon l'invention permet par ailleurs d'assurer le suivi de traitements thérapeutiques, notamment de traitements coagulants ou anticoagulants, administrés à des individus présentant une anomalie de la coagulation. De tels traitements sont désignés ci-après comme étant des « traitement d'une anomalie de la coagulation sanguine ».

De tels traitements coagulants sont administrés, souvent de façon prolongée, chez les individus présentant une anomalie de la coagulation.

En particulier, la présente invention se rapporte à une méthode pour déterminer l'efficacité d'un traitement d'une anomalie de la coagulation sanguine chez un individu, comprenant les étapes suivantes :

i) déterminer la valeur de l'aire sous la courbe d'un échantillon de sang dudit individu avant le début dudit traitement, en effectuant les étapes (a) (b) (c) et (d) de la méthode décrite précédemment, et

ii) déterminer la valeur de l'aire sous la courbe d'un échantillon de sang dudit individu après le début dudit traitement, en effectuant les étapes (a) (b) (c) et (d) de la méthode décrite précédemment,

ledit traitement étant jugé comme efficace lorsque la valeur de l'aire sous la courbe déterminée à l'étape ii) est supérieure à la valeur de l'aire sous la courbe déterminée à l'étape i).

Selon un aspect particulier de l'invention, ladite méthode pour déterminer l'efficacité d'un traitement d'un état hémophile d'un individu, comprend les étapes suivantes :

i) déterminer la valeur de l'aire sous la courbe d'un échantillon de sang dudit individu à un instant $T_1$ après le début dudit traitement, en effectuant les étapes (a) (b) (c) et (d) de la méthode décrite précédemment, et

ii) déterminer la valeur de l'aire sous la courbe d'un échantillon de sang dudit individu à un instant $T_2$, postérieur au premier instant $T_1$, en effectuant les étapes (a) (b) (c) et (d) de la méthode décrite précédemment,

ledit traitement étant jugé comme efficace lorsque la valeur de l'aire sous la courbe déterminée à l'étape ii) est supérieure à la valeur de l'aire sous la courbe déterminée à l'étape i).

Enfin, la présente demande concerne, sans que cet aspect ne fasse partie de l'invention, une méthode pour cribler des molécules thérapeutiques susceptibles d'agir sur le processus de coagulation, comprenant les étapes suivantes :

i) obtenir deux échantillons de sang d'un même individu traité avec une molécule thérapeutique candidate, l'un avant le début du traitement, et l'autre pendant ou après le traitement;

ii) déterminer la valeur de l'aire sous la courbe de l'échantillon de sang obtenu avant le début dudit traitement, en effectuant les étapes (a) (b) (c) et (d) de la méthode décrite précédemment, et

iii) déterminer la valeur de l'aire sous la courbe de l'échantillon de sang obtenu pendant ou après ledit traitement, en effectuant les étapes (a) (b) (c) et (d) de la méthode décrite précédemment,

iv) comparer les valeurs obtenues aux étapes (ii) et (iii) pour déterminer l'effet du traitement avec ladite molécule thérapeutique.

## EXEMPLES

### Exemple 1. Protocole expérimental

300 microlitres d'une solution de NaCl/CaCl$_2$ (Roche Diagnostic) sont placés dans la cupule, avant l'ajout de 300 micro litres de sang prélevé sur tube citraté 0.107 M ou 0.109 M. La cupule est munie d'un barreau aimanté pour l'agitation et de deux paires d'électrodes en argent connecté à l'automate Multiplate® (Roche diagnostic).

Après 120 secondes d'incubation, la coagulation est déclenchée par ajout d'une solution de facteur tissulaire, diluée dans du tampon Hepes; la concentration finale dans l'échantillon est de 2 pM (figures 1 à 4), de 100 pM (figure 6A, 6B) ou de 1 pM (figure 7A, 7B).

La mesure de la variation d'impédance entre les électrodes (exprimée en unités arbitraires - UA) est réalisée sur une période de 20 minutes. Une courbe est ainsi générée, en fonction du temps, et la courbe ainsi tracée définit une aire sous la courbe, mesurée en UA*minutes (indiquée dans les figures 'AUC').

**Exemple 2. Résultats obtenus avec l'ajout de facteur tissulaire à une concentration de 2 pM**

Les figures 1 à 4 montrent, respectivement, que :

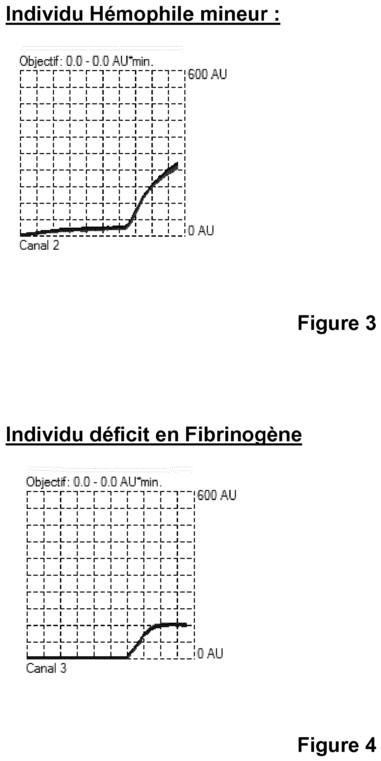

- la courbe obtenue avec un échantillon de sang issu d'un individu ne présentant pas de troubles de la coagulation ; l'aire sous la courbe est de 4703 UA*minutes (Fig 1.)
- la courbe obtenue avec un échantillon de sang issu d'un individu diagnostiqué comme étant un hémophile sévère ; l'aire sous la courbe est de 301 UA*minutes. (Fig 2.)
- la courbe obtenue avec un échantillon de sang issu d'un individu diagnostiqué comme présentant une hémophilie mineure ; l'aire sous la courbe est de 2180 UA*minutes. (Fig 3.)
- la courbe obtenue avec un échantillon de sang issu d'un individu diagnostiqué comme présentant un déficit en fibrinogène ; l'aire sous la courbe est de 1173 UA*minutes. (Fig 4.)

Il peut ainsi être déduit de ces courbes que lorsque l'aire sous la courbe est inférieure à une valeur seuil dite « de référence », une anomalie de la coagulation est présente.

La figure 5 représente la répartition des valeurs d'aires sous la courbe, en fonction des caractéristiques des individus testés.

Dans le cas présent, une valeur seuil de 4000 unités arbitraires *minutes (AUC) a pu être déterminée, qui sépare :

- les valeurs autour de 6000 AUC caractéristiques des individus témoins, ne présentant pas d'anomalies de la coagulation ; et
- les valeurs inférieures ou égales à 4000 AUC, caractéristiques des individus présentant une anomalie de la coagulation, même mineure ; parmi ces individus, ceux présentant une anomalie sévère présentent des valeurs sous la courbe inférieures à 2000 AUC.

La figure 13 a été réalisée selon le même protocole expérimental que la figure 5, les seules modifications étant l'addition de 1 pM de facteur tissulaire dans le milieu réactionnel, et le nombre plus importants d'échantillons de sang testés.

Dans cette expérience, deux échantillons de sang d'individus atteints d'un déficit en facteur VII ont pu être testés. Les résultats sont concordants avec ceux présentés en figure 5.

Sur ces figures, chaque valeur individuelle d'AUC est représentée par un point ; la moyenne des valeurs obtenues dans chaque groupe d'individus est représentée par un trait horizontal ; deux autres traits plus courts, au-dessus et en-dessous de la moyenne, représentent l'écart type observé autour de cette moyenne de valeurs.

**Exemple 3. Ajustement de la concentration nécessaire de facteur tissulaire pour déclencher la coagulation**

Des expériences ont été réalisées, selon le même protocole que celui décrit en exemple 1, pour déterminer une gamme de concentration optimale de facteur tissulaire adéquate pour déclencher la coagulation sanguine *in vitro,* dans le cadre du test décrit dans la présente demande.

Deux concentrations ont été testées : 100 pM et 1 pM, sur deux types d'échantillons sanguins : l'un provenant d'un individu sain ne présentant pas d'anomalie de la coagulation (A), l'autre provenant d'un sujet hémophile modéré.

Lorsque la coagulation est déclenchée avec une concentration de 100 pM de facteur tissulaire, les valeurs d'aires sous la courbe sont élevées, et distinguables entre les deux individus :

- 17 191 UA*minute pour l'individu sain ne présentant pas de trouble de la coagulation ;
- 11 638 UA*minute pour l'individu hémophile, (figures 6A, 6B)

Lorsque la coagulation est déclenchée avec une concentration de 1 pM de facteur tissulaire, les valeurs d'aires sous la courbe sont moins élevées, et sont plus nettement distinguables entre les deux individus :

- 4 838 UA*minute pour l'individu sain ne présentant pas de trouble de la coagulation;
- 524 UA*minute pour l'individu hémophile, (figures 7A, 7B)

Avec cette concentration en facteur tissulaire, la différence de valeur entre les deux sujets est bien nette, l'individu hémophile présentant une valeur d'aire sous la courbe inférieure à 15% de la valeur de l'aire sous la courbe de l'individu sain, cette courbe étant ici la courbe de référence.

**Exemple 4. Une corrélation directe existe entre le taux de facteur VIII et la valeur d'aire sous la courbe**

La figure 8 montre une courbe linéaire de la corrélation observée entre le taux d'activité du facteur VIII plasmatique (en abscisse) et l'aire sous la courbe mesurée chez les individus (en ordonnées). Le coefficient de corrélation r2 est excellent : r2=0,883.

Le taux d'activité du facteur VIII a été mesuré par une technique chronométrique sur un automate de coagulation, dont le principe repose sur la mesure d'un temps de coagulation (TCA) d'un mélange constitué de plasma du patient et de plasma déficient en facteur VIII.

Il est bien connu que le taux d'activité du facteur VIII plasmatique est inférieur à 1% pour les hémophiles A sévères, compris entre 1 et 5% pour les hémophiles A modérés, et compris entre 5 et 40% pour les hémophiles A mineurs. (White GC II *et al.,* 2001). Les valeurs au-dessus de 40% ont été déterminées sur des échantillons d'individus non hémophiles.

Comme démontré précédemment, la valeur de l'aire sous la courbe est dépendante de la sévérité de l'anomalie de la coagulation.

En rapportant le taux d'activité du facteur VIII avec la valeur d'aire sous la courbe, on a pu constater qu'il existe une relation directe entre le taux d'activité du facteur de coagulation VIII et la valeur d'aire sous la courbe mesurée par la méthode selon l'invention.

**Exemple 5. Confirmation des résultats sur des cohortes d'individus plus larges**

Le même protocole expérimental que celui présenté dans l'exemple 1 a été réalisé, avec addition de 1 pM de facteur tissulaire dans le milieu réactionnel. Les tests réalisés précédemment sur de petits effectifs ont été reproduits sur de plus larges cohortes d'individus. La valeur seuil d'aire sous la courbe de 4000 AUC a été confirmée sur ces larges cohortes.

La figure 9 met en évidence la pertinence de cette valeur seuil de 4000 AUC sur des échantillons de 23 individus sains, et de 42 individus atteints d'hémophilie, tout type d'hémophilie et toutes sévérités confondues : tous les individus hémophiles présentent des valeurs AUC situées en dessous de la courbe seuil tracée à 4000 AUC.

La valeur moyenne d'aire sous la courbe des individus sains (témoins) est de 5930, alors que la valeur moyenne des aires sous la courbe obtenues à partir des échantillons d'individus hémophiles est de 1192. L'analyse statistique de la différence observée entre les valeurs des échantillons d'individus sains et des individus hémophiles est très significative (p<0,0001).

**Exemple 6. Diagnostic de l'hémophilie A par le procédé selon l'invention**

Le protocole expérimental de l'exemple 1, avec une concentration finale de 1 pM de facteur tissulaire, a été réalisé pour distinguer des individus hémophiles A, présentant plusieurs niveaux de sévérité de la maladie.

Les individus hémophiles A sont classifiés ainsi :

- Hémophilie A sévère : le taux d'activité de Facteur VIII est inférieur à 1% de l'activité normale ;
- Hémophilie A modérée : le taux d'activité de Facteur VIII est compris entre 1% et 5% ;
- Hémophilie A mineure : le taux d'activité de Facteur VIII est compris entre 5% et 40%.

Les résultats individuels obtenus sont présentés dans la figure 10, et les moyennes dans le tableau 3 ci-dessous :

**Tableau 3**

|  | Individus témoins | Hémophiles sévères | Hémophiles modérés | Hémophiles mineurs |
|---|---|---|---|---|
| Moyenne des AUC | 5930 | 426 | 840 | 1861 |
| Effectif du groupe testé | 23 | 9 | 10 | 8 |

**Exemple 7. Diagnostic de l'hémophilie B par le procédé selon l'invention**

Le protocole expérimental de l'exemple 1, avec une concentration finale de 1 pM de facteur tissulaire, a été réalisé pour distinguer des individus hémophiles B, présentant plusieurs niveaux de sévérité de la maladie.

Les individus hémophiles B sont classifiés ainsi :

- Hémophilie B sévère : le taux de Facteur IX est inférieur à 1% du taux d'activité normalement observé, en l'absence d'anomalie de la coagulation ;
- Hémophilie B modérée : le taux de Facteurs IX est compris entre 1% et 5% ;
- Hémophilie B mineure : le taux de Facteur IX est compris entre 5% et 40%.

Les résultats individuels obtenus sont présentés dans la figure 11, et les moyennes dans le tableau 4 ci-dessous :

**Tableau 4**

|  | Individus témoins | Hémophiles sévères | Hémophiles modérés | Hémophiles mineurs |
|---|---|---|---|---|
| Moyenne des AUC | 5930 | 768 | 938 | 2669 |
| Effectif du groupe testé | 23 | 3 | 7 | 3 |

**Exemple 8. Corrélation directe entre le taux de facteur IX et la valeur d'aire sous la courbe**

La figure 12 montre une courbe linéaire de la corrélation observée entre le taux d'activité du facteur IX plasmatique (en abscisse) et l'aire sous la courbe mesurée chez les individus (en ordonnées).
Le taux d'activité du facteur IX a été mesuré par une technique chronométrique sur un automate de coagulation, dont le principe repose sur la mesure d'un temps de coagulation (TCA) d'un mélange constitué de plasma du patient et de plasma déficient en facteur IX.
Il est bien connu que le taux d'activité du facteur IX est inférieur à 1% pour les hémophiles sévères, compris entre 1 et 5% pour les hémophiles modérés, et compris entre 5 et 40% pour les hémophiles mineurs. (White GC II *et al.,* 2001). Les valeurs au-dessus de 40% ont été déterminées sur des échantillons d'individus non hémophiles.
Comme démontré précédemment, la valeur de l'aire sous la courbe est dépendante de la sévérité de l'anomalie de la coagulation.

**REFERENCES BIBLIOGRAPHIQUES**

US 8,877,510

EP 2 182 345

Lang T, Bauters A, Braun SL, et al. Multi-centre investigation on reference ranges for ROTEM thromboelastometry. Blood Coagul Fibrinolysis 2005 ; 16 : 301-10.

White GC II, Rosendaal F, Aledort LM, Lusher JM, Rothschild C, Ingerslev J. Definitions in hemophilia. Recommendation of the scientific subcommittee on factor VIII and factor IX of the scientific and standardization committee of the International Society on Thrombosis and Haemostasis. Thromb Haemost. 2001 Mar;85(3):560

**Revendications**

1.    Méthode pour diagnostiquer des anomalies de la coagulation sanguine, comprenant les étapes successives suivantes :

a) placer un échantillon de sang total dans une cuve contenant deux paires d'électrodes connectées à un appareil générant un courant électrique ;
b) incuber cet échantillon pendant 60 à 180 secondes en présence de calcium ;
c) ajouter à cet échantillon du facteur tissulaire en une concentration adéquate pour déclencher la coagulation sanguine ;
d) mesurer la variation d'impédance entre les électrodes en fonction du temps, pendant une période comprise entre 10 et 30 minutes à partir de l'étape (c) et générer une courbe des valeurs d'impédance en fonction du temps;
e) comparer la valeur de l'aire sous la courbe générée à l'étape (d) avec une valeur d'aire sous la courbe de référence.

2.    Méthode selon la revendication 1, **caractérisée en ce que** la mesure de la variation d'impédance est réalisée pendant une période d'environ 20 minutes.

3. Méthode selon l'une des revendications 1 à 2, **caractérisée en ce que** l'échantillon de sang total est citraté.

4. Méthode selon l'une des revendications 1 à 3, **caractérisée en ce que** le facteur tissulaire est ajouté à une concentration finale dans l'échantillon comprise entre 0.5 et 5 pM.

5. Méthode selon l'une des revendications 1 à 4, **caractérisée en ce que** le volume de l'échantillon de sang total est inférieur à 1 ml, de préférence inférieur à 500 $\mu$l.

6. Méthode selon l'une des revendications 1 à 5, **caractérisée en ce qu'**à l'étape (b) une solution saline comprenant du calcium est ajoutée à l'échantillon, de préférence en effectuant une dilution volume / volume.

7. Méthode selon l'une des revendications 1 à 6, **caractérisée en ce que** la courbe de référence est une courbe moyenne obtenue en réalisant les étapes (a) à (d) sur des échantillons de sang issus d'individus ne présentant pas d'anomalie de la coagulation.

8. Méthode selon l'une des revendications 1 à 7, **caractérisée en ce que** la variation d'impédance est mesurée une fois par minute pour générer la courbe.

9. Méthode selon l'une des revendications 1 à 8, **caractérisée en ce que** les anomalies de la coagulation diagnostiquées sont des troubles de l'hémostase secondaire, en particulier choisis parmi les coagulopathies constitutionnelles et les coagulopathies acquises.

10. Méthode selon la revendication 9, **caractérisée en ce que** les anomalies de la coagulation diagnostiquées sont :

> - des coagulopathies constitutionnelles choisies parmi l'Hémophilie A ou B, et le déficit en facteurs de la coagulation ; ou
> - des coagulopathies acquises dues à la prise médicamenteuse d'inhibiteurs de la coagulation.

11. Méthode selon la revendication précédente, **caractérisée en ce que** l'anomalie de la coagulation diagnostiquée est un déficit en facteur XI, VII, XIII, X, V, II ou en fibrinogène.

12. Méthode selon la revendication 1, **caractérisée en ce que** ladite méthode est pour déterminer la sévérité d'une anomalie de la coagulation.

13. Méthode pour déterminer l'efficacité d'un traitement d'une anomalie de la coagulation sanguine chez un individu, comprenant les étapes suivantes :

> i) déterminer la valeur de l'aire sous la courbe d'un échantillon de sang dudit individu avant le début dudit traitement, en effectuant les étapes (a) (b) (c) et (d) de la méthode selon l'une des revendications 1 à 12, et
> ii) déterminer la valeur de l'aire sous la courbe d'un échantillon de sang dudit individu après le début dudit traitement, en effectuant les étapes (a) (b) (c) et (d) de la méthode selon l'une des revendications 1 à 12,

ledit traitement étant jugé comme efficace lorsque la valeur de l'aire sous la courbe déterminée à l'étape ii) est supérieure à la valeur de l'aire sous la courbe déterminée à l'étape i).

**Patentansprüche**

1. Verfahren zur Diagnose von Anomalien der Blutgerinnung, umfassend die folgenden aufeinander folgenden Schritte:

> a) Überführen einer Vollblutprobe in eine Küvette, die zwei an eine elektrischen Strom erzeugende Apparatur angeschlossene Elektrodenpaare enthält;
> b) Inkubieren dieser Probe 60 bis 180 Sekunden lang in Gegenwart von Calcium;
> c) Zugeben von Gewebefaktor zu dieser Probe in einer zum Auslösen der Blutgerinnung ausreichenden Konzentration;
> d) Messen der Impedanzveränderung zwischen den Elektroden in Abhängigkeit von der Zeit während eines Zeitraums zwischen 10 und 30 Minuten ab Schritt (c) und Erstellen einer Kurve der Impedanzwerte in Abhängigkeit von der Zeit;

e) Vergleichen des Werts für die Fläche unter der in Schritt (d) erstellten Kurve mit einem Wert für die Fläche unter der Referenzkurve.

**2.** Verfahren nach Anspruch 1, **dadurch gekennzeichnet, dass** die Messung der Impedanzveränderung über einen Zeitraum von etwa 20 Minuten durchgeführt wird.

**3.** Verfahren nach einem der Ansprüche 1 bis 2, **dadurch gekennzeichnet, dass** die Vollblutprobe Citratblut ist.

**4.** Verfahren nach einem der Ansprüche 1 bis 3, **dadurch gekennzeichnet, dass** der Gewebefaktor in einer Endkonzentration in der Probe zwischen 0,5 und 5 pM zugegeben wird.

**5.** Verfahren nach einem der Ansprüche 1 bis 4, **dadurch gekennzeichnet, dass** das Volumen der Vollblutprobe kleiner als 1 ml, vorzugsweise kleiner als 500 µl ist.

**6.** Verfahren nach einem der Ansprüche 1 bis 5, **dadurch gekennzeichnet, dass** in Schritt (b) eine Calcium enthaltende Kochsalzlösung, vorzugsweise mittels Durchführen einer Volumen/Volumen-Verdünnung, zu der Probe zugegeben wird.

**7.** Verfahren nach einem der Ansprüche 1 bis 6, **dadurch gekennzeichnet, dass** die Referenzkurve eine Durchschnittskurve ist, die mittels Durchführen der Schritte (a) bis (d) an Blutproben von Individuen, die keine Gerinnungsanomalie zeigen, erhalten wird.

**8.** Verfahren nach einem der Ansprüche 1 bis 7, **dadurch gekennzeichnet, dass** zur Erstellung der Kurve die Impedanzveränderung einmal pro Minute gemessen wird.

**9.** Verfahren nach einem der Ansprüche 1 bis 8, **dadurch gekennzeichnet, dass** die diagnostizierten Gerinnungsanomalien Störungen der sekundären Hämostase sind, die insbesondere aus angeborenen Koagulopathien und erworbenen Koagulopathien ausgewählt sind.

**10.** Verfahren nach Anspruch 9, **dadurch gekennzeichnet, dass** die diagnostizierten Gerinnungsanomalien Folgende sind:

- angeborene Koagulopathien, ausgewählt aus Hämophilie A oder B und Mangel an Gerinnungsfaktoren, oder
- erworbene Koagulopathien aufgrund der Gabe gerinnungshemmender Medikamente.

**11.** Verfahren nach dem vorhergehenden Anspruch, **dadurch gekennzeichnet, dass** die diagnostizierte Gerinnungsanomalie ein Mangel an Faktor XI, VII, XIII, X, V, II oder an Fibrinogen ist.

**12.** Verfahren nach Anspruch 1, **dadurch gekennzeichnet, dass** das Verfahren zur Bestimmung der Schwere einer Gerinnungsanomalie dient.

**13.** Verfahren zum Bestimmen der Wirksamkeit einer Behandlung einer Blutgerinnungsanomalie bei einem Individuum, umfassend die folgenden Schritte:

i) Bestimmen des Werts der Fläche unter der Kurve einer Blutprobe von dem Individuum vor Beginn der Behandlung mittels Durchführen der Schritte (a) (b) (c) und (d) des Verfahrens nach einem der Ansprüche 1 bis 12 und

ii) Bestimmen des Werts der Fläche unter der Kurve einer Blutprobe von dem Individuum nach Beginn der Behandlung mittels Durchführen der Schritte (a) (b) (c) und (d) des Verfahrens nach einem der Ansprüche 1 bis 12,

wobei die Behandlung als wirksam erachtet wird, wenn der in Schritt ii) ermittelte Wert für die Fläche unter der Kurve größer ist als der in Schritt i) ermittelte Wert für die Fläche unter der Kurve.

**Claims**

1. Method for diagnosing blood coagulation abnormalities, comprising the following successive steps:

   a) placing a whole blood sample in a receptacle containing two pairs of electrodes connected to an apparatus generating an electric current;
   b) incubating this sample for 60 to 180 seconds in the presence of calcium;
   c) adding, to this sample, tissue factor in a concentration sufficient for triggering blood coagulation;
   d) measuring the variation in impedance between the electrodes as a function of time for a period of between 10 and 30 minutes starting from step (c) and generating a curve of the impedance values as a function of time;
   e) comparing the value of the area under the curve generated in step (d) with a value of an area under the reference curve.

2. Method according to Claim 1, **characterized in that** the measurement of the variation in impedance is carried out for a period of about 20 minutes.

3. Method according to either of Claims 1 and 2, **characterized in that** the whole blood sample is citrated.

4. Method according to one of Claims 1 to 3, **characterized in that** the tissue factor is added to a final concentration in the sample of between 0.5 and 5 pM.

5. Method according to one of Claims 1 to 4, **characterized in that** the volume of the whole blood sample is less than 1 ml, and preferably less than 500 µl.

6. Method according to one of Claims 1 to 5, **characterized in that**, in step (b), a saline solution comprising calcium is added to the sample, preferably by effecting a volume/volume dilution.

7. Method according to one of Claims 1 to 6, **characterized in that** the reference curve is a mean curve obtained by carrying out steps (a) to (d) on blood samples obtained from individuals not exhibiting a coagulation abnormality.

8. Method according to one of Claims 1 to 7, **characterized in that** the variation in impedance is measured once per minute in order to generate the curve.

9. Method according to one of Claims 1 to 8, **characterized in that** the coagulation abnormalities diagnosed are disorders of secondary haemostasis, in particular selected from constitutional coagulopathies and acquired coagulopathies.

10. Method according to Claim 9, **characterized in that** the coagulation abnormalities diagnosed are:

    - constitutional coagulopathies selected from haemophilia A or B, and coagulation factor deficiency; or
    - acquired coagulopathies caused by the medicinal taking of coagulation inhibitors.

11. Method according to the preceding claim, **characterized in that** the coagulation abnormality diagnosed is a factor XI, VII, XIII, X, V, II or fibrinogen deficiency.

12. Method according to Claim 1, **characterized in that** said method is for determining the severity of a coagulation abnormality.

13. Method for determining the efficacy of a treatment of a blood coagulation abnormality in an individual, comprising the following steps:

    i) determining the value of the area under the curve for a blood sample from said individual before the beginning of said treatment, by carrying out steps (a), (b), (c) and (d) of the method according to one of Claims 1 to 12, and
    ii) determining the value of the area under the curve for a blood sample from said individual after the beginning of said treatment, by carrying out steps (a), (b), (c) and (d) of the method according to one of Claims 1 to 12,

    said treatment being considered effective when the value of the area under the curve determined in step ii) is greater than the value of the area under the curve determined in step i).

## Individu normal :

Objectif: 0.0 - 0.0 AU*min.

600 AU

0 AU

Canal 1

**Figure 1**

## Individu Hémophile sévère :

Objectif: 0.0 - 0.0 AU*min.

400 AU

0 AU

Canal 1

**Figure 2**

## Individu Hémophile mineur :

Objectif: 0.0 - 0.0 AU*min.

600 AU

0 AU

Canal 2

## Figure 3

## Individu déficit en Fibrinogène

Objectif: 0.0 - 0.0 AU*min.

600 AU

0 AU

Canal 3

## Figure 4

**Figure 5**

## Individu sain :

Objectif: 0.0 - 0.0 AU*min.
600 AU
0 AU
Canal 1

**Figure 6A**

## Individu Hémophile :

Objectif: 0.0 - 0.0 AU*min.
600 AU
0 AU
Canal 1

**Figure 6B**

## Individu sain :

Objectif: 0.0 - 0.0 AU*min.

600 AU

0 AU

Canal 1

## Figure 7A

## Individu hémophile :

Objectif: 0.0 - 0.0 AU*min.

400 AU

0 AU

Canal 2

## Figure 7B

## Corrélation AUC et taux de Facteur VIII

## Figure 8

**Hémophilie**

## Figure 9

## Hemophilie A

**Figure 10**

## Hemophilie B

**Figure 11**

## corrélation AUC et taux de facteur IX

**Figure 12**

**Figure 13**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 8877510 B

- EP 2182345 A

**Littérature non-brevet citée dans la description**

- **LANG T** ; **BAUTERS A** ; **BRAUN SL et al.** Multicentre investigation on reference ranges for ROTEM thromboelastometry. *Blood Coagul Fibrinolysis*, 2005, vol. 16, 301-10

- **WHITE GC II** ; **ROSENDAAL F** ; **ALEDORT LM** ; **LUSHER JM** ; **ROTHSCHILD C** ; **INGERSLEV J**. Definitions in hemophilia. Recommendation of the scientific subcommittee on factor VIII and factor IX of the scientific and standardization committee of the International Society on Thrombosis and Haemostasis. *Thromb Haemost*, March 2001, vol. 85 (3), 560